# EUROPEAN PATENT SPECIFICATION

(11) **EP 1 649 043 B1**
(45) Date of publication and mention of the grant of the patent: **15.12.2010**
(21) Application number: 04766336.4
(22) Date of filing: 27.07.2004
(51) Int. Cl.: C12Q 1/04, G01N 33/569

(54) **METHOD FOR THE DETECTION OF COLIFORMS AND IN PARTICULAR ESCHERICHIA COLI**
VERFAHREN ZUM NACHWEIS VON COLIFORMEN UND INSBESONDERE ESCHERICHIA COLI
PROCEDE DE DETECTION DE COLIFORMES, EN PARTICULIER D' ESCHERICHIA COLI

(30) Priority: 31.07.2003 IT TR20030002
(43) Date of publication of application: 26.04.2006
(73) Proprietor: ISRIM S. CONS. A.R.L., 05100 Terni (IT)
(72) Inventor: BODINI, Sergio, I-20013 Magenta (IT); SANTORI, Francesca, I-05100 Terni (IT)
(74) Representative: Jorio, Paolo
(86) International application number: PCT/EP2004/051626
(87) International publication number: WO 2005/012555

(56) References cited:
- WO-A-97/49830
- US-A- 5 861 270
- US-A- 5 935 799
- US-B1- 6 329 166
- BERG J D ET AL: "RAPID DETECTION OF TOTAL AND FECAL COLIFORMS IN WATER BY ENZYMATIC HYDROLYSIS OF 4 METHYLUMBELLIFERONE-BETA-D-GALACTOSIDE" APPLIED AND ENVIRONMENTAL MICROBIOLOGY, vol. 54, no. 8, 1988, pages 2118-2122, XP002304253 ISSN: 0099-2240

## Description

### Technical Field

The present invention relates to a method for detecting coliforms which employs an enzyme-inducing solution containing one or more amino acids, capable to promote the expression of inducible enzymes in absence of cell growth. This method can be used for the rapid detection of a very small number of *E*. *coli* and/or total coliforms, by measuring enzymatic activity of the enzymes β-glucuronidase and β-galactosidase.

### Background Art

Many rapid methods for the identification/detection of microorganisms are based on the presence of enzymes involved in specific metabolic activities. These enzymes can be constitutive, which means always expressed, or inducible, namely present only in determined metabolic conditions. That is the case of the enzymes β-glucuronidase and β-galactosidase expressed in coliform bacteria in presence of a specific inducer.

Coliform bacteria are "indicator microbes" of the presence of traces of human sewage. They are commonly divided in total coliforms and faecal coliforms, including the former also coliforms found in surface soil and water, and the latter, the most common member being *E. coli,* only gram-negative thermo resistant bacteria, naturally inhabiting both human and animal gastrointestinal tract, causing, thus, urogenital infections and diarrhea.

Total coliforms are characterized by the possession of the gene coding for the enzyme β-galactosidase, responsible for the hydrolysis of lactose to galactose and glucose. The determination of β-galactosidase can be achieved by means of chromogenic and/or fluorogenic substrates.

β-glucuronidase is an enzyme which catalyzes the hydrolysis of β-D-glucopyranosiduronic acids into the correspondent aglycons and D-glucuronic acid; like β-galactosidase, the activity is measured by using different chromogenic and/or fluorogenic substrates. It is present in the 94-96% [Acta Pathol. Microbiol. Scand. Sect. B, 84:245(1976)] of different types of *E*. *coli.* It is therefore specifically used for the detection of *E*. *coli.*

The production of β-galactosidase can be enhanced by using the "natural" inducer lactose or non-metabolizable analogues, like methyl-β-D-thiogalactopyranoside (MTG) and isopropyl-β-D-thiogalactopyranoside (IPTG) that, not releasing the glucosidic part, do not cause the interruption of the enzymatic synthesis. The synthesis of β-glucuronidase may be similarly stimulated by methyl-β-D-glucuronide or isopropyl-β-D-glucuronide. Literature data show that, up to today, the methods for detecting the expression of those enzymes are based on the enzymatic induction achieved by means of specific growth media, where the production of β-galactosidase/β-glucuronidase is promoted through a process of cell duplication. Carbon sources (like lactose), nitrogen sources, vitamins and mineral salts are usually contained in common culture media, in order to assist the desired growth.

Berg and Fiksdal disclose (patents US55188894, US5292644, EP0574977 and WO8904372) the use of a nutritive growth media, selective for coliforms, containing lactose as a production agent for β-galactosidase, yet enriched with the fluorogenic enzyme substrate methylumbelliferyl-β-D-galactoside (MUGal), providing thus a fluorimetric response after not more than 8 hours of incubation. The evolution of this process leads to the development of a kit for rapid detection of coliforms known as Colifast™ (patents US55188894, US5292644, EP0574977 and WO8904372). Using this system, Tryland et al. [Wat. Sci. Tech., Vol.43 N°12 217-220 (2001)] and Farnleitner et al. [Lett. Appl. Microbiol. Vol.33 Iss.3 246-250 (2001)] verify, respectively, a highly significant relationship between β-galactosidase activity and coliforms count, and between the rate of β-glucuronidase hydrolysis and *E. coli* concentration. This method, compared to the method of the current invention, presents the following disadvantages:
1. the cell duplication introduces another variable influencing the final result; besides the enzyme produced at the single cell level, also the parameter of the final cell count must be taken into account;
2. the detection time, especially when a number of cells <1000 is present in the sample, is strongly conditioned from the need to quantitate in the exponential phase; time proposed from the Applicant is shorter.

Edberg (US 4925789, 1990) discloses the concept of "nutrient-indicator". The growth medium includes, as a primary carbon source, a specific nutrient, modified by attaching a sample-altering moiety thereto, which only the target microbe can metabolize. The moiety is activated to alter the sample only if the nutrient is metabolized by the target microbe, defining therefore its presence/absence. Edberg specifically detects *E. coli,* using a chromogenic or fluorogenic substrate, selected among o-nitrophenyl-β-D-glucuronide (ONPG, yellow), β-naphtalamide-β-D-glucuronide (purple), α-naphtol-β-D-glucuronide (red), methylumbelliferyl-β-D-glucuronide (MUGlu, fluorescent). He also tests combinations among them for their ability to determine simultaneously *E. coli* and total coliforms, by shuffling different substrates for β-glucuronidase (*E*. *coli*) and for β-galactosidase (coliforms). The incubation times vary between 18 and 22 hours. The product Colilert™ and the MMO/MUG test, approved from the U.S. Environmental Protection Agency (USEPA), where ONPG is used for total coliforms and MUGlu for *E*. *coli,* are based on the general principles disclosed by Edberg. This procedure requires at least 18 hours to complete. Time proposed from the Applicant is shorter.

Roth et al. (US5210022, 1993) disclose test media and chromogenic compounds for identifying and differentiating *E*. *coli* and general coliforms. The test medium is formed by combining a nutrient base medium with a chromogenic substrate for β-galactosidase, which causes the formation of an insoluble precipitate of a first colour upon reacting with the enzyme β-gal, and also a chromogenic substrate for β-glucuronidase, capable to generate an insoluble precipitate of a second colour contrasting with the first colour upon reacting with the enzyme GUD. From this disclosure takes place the commercial product Colichrome 2™. The method requires 24-48 hours incubation. Time proposed from the Applicant is shorter.

Brenner et al. [Appl. Environ. Microbiol.1993 Nov; 59(11):3534-44] provide the improved medium MIagar, incorporating methylumbelliferyl-β-D-galactoside (MUGal) as the β-galactosidase substrate to identify total coliforms, and indoxyl-β-D-glucuronide (Ibdg) for detecting *E*. *coli* through the β-glucuronidase activity (US6063590. 2000). This method, also intended as a cell growth procedure, takes at least 20 hours. Time proposed from the Applicant is shorter.

In an article Van Poucke and Nelis [Appl. Environ. Microbiol. Vol.63 N°2 771-774 (1997)] describe a chemiluminometric test for the detection of total coliforms and *E*. *coli* in a liquid medium. The new medium Colicult™ allows to reduce detection time to 6 hours for total coliforms, being Galacton-Plus enzymatic substrate and inducer, and 9 hours for *E*. *coli,* being Glucuron substrate and inducer (US5861270, 1999). This method, like already explained when treating the procedure disclosed by Berg and Fiksdal, suffers disadvantages due to cell duplication and long detection time (6-9 hours), because of the need to quantitate in the exponential phase of cell growth. The method proposed from the Applicant provides a shorter detection time and cell growth is not required.

Grant (US5849515, 1998) discloses a culture medium which permits simultaneous detection of total coliforms and *E*. *coli,* by using a selective β-glucuronidase substrate, like the salt cyclohexylammonium 5-bromo-4-chloro-3-indolyl-β-D-glucuronate (X-Gluc), and a highlighting dye, like triphenyltetrazolium chloride, for enhancing visual differentiation in the detection of β-galactosidase. Originated from this disclosure is the product m-Coliblue24. In the composition of the growth medium, lactose is both carbon source and enzymatic inducer, and some essential amino acids are contrast promoters for increasing the formation of β-glucuronidase by *E*. *coli.* Even though the importance of some amino acids is evidenced, the spirit of the work is still definitely directed to a cell growth system leading to a cfu (colony forming units) count. The test lasts therefore 36-48 hr. Edberg (US6329166) discloses a composition for detecting the presence or absence of target microbes in an environmental or biological sample. The composition comprises ingredients such as Ammonium Sulfate, Vitamins and Sugars, that are necessary for the microbiological growth.

The Applicant is able, in any case, to raise at the maximum rate, the enzymatic synthesis at the single cell level, independently from the environmental conditions from which the bacteria happen to be sampled; it is actually impossible a definition a priori of optimal introduction", being the enzymatic profile strongly dependent from the outer environment.

In conclusion:
1. methods based on cells count an Petri dishes imply analysis times exceeding 12 hours;
2. methods based an a direct analysis in liquid medium also need to quantitate in the exponential phase of cell growth, resulting in slow detection times; another variable, concerning the cell growth, must be taken into account when defining a linear correlation between the enzymatic activity and the original number of cells;
3. methods based on the presumption that, in determinate environmental circumstances, the enzymatic activity of coliform cells is naturally induced, limit the universal feasibility of the process, subject the results to false negatives, and turn out to be strictly dependent from the level of enzymatic induction present at the moment of sampling.

The current invention discloses a sensitive and rapid method for the detection of coliforms, performed in absence of cell growth, which overcomes the above-mentioned deficiencies and disadvantages.

The method hereby described is based on the simple observation that the amino acids, singularity or in a mixture, are capable of sensibly incrementing the expression of β-galactosidase and β-glucuronidase in the single coliform cell, through a process not requiring cell duplication; this occurence allows to achieve a detectable amount of enzymes, even when dealing with a very poor number of cells.

### Disclosure of Invention

The object of the current invention is an induction solution, the essential characteristics of which are specified in Claim 1, and preferred and/or auxiliary characteristics of which are specified in Claims 2-7.

Another aspect of the invention concerns a method for detection of microorganisms, preferably in water samples or any or any other sample in which the microbes can be extracted, for analytical purposes, in liquid environment.

Yet another aspect of the invention is a method allowing the determination of the concentration of total coliforms or *E*. *coli* in waste water, surface water, bathing water, fresh water, sea water and urban water. Solid samples like food, soil, etc., requiring a microbiological investigation, may be analysed with this method after opportune extraction in liquid phase.

According to necessity the sample can be:
- directly added to the induction solution;
- filtered to concentrate bacteria on a membrane filter with a pore size not superior to 0.45 µm, then the membrane filter and the bacteria concentrated thereon is immersed in a minimum volume of induction solution, enough to cover up, the retained material;
- extracted with suitable liquid medium, filtered to concentrate bacteria on a membrane filter with a pore size not superior to 0.45 µm, then the membrane filter and the bacteria concentrated thereon are immersed in a minimum volume of induction solution, enough to cover up the retained material.

The induction step is performed between 30°C and 40°C for total coliforms and between 30°C and 50°C for faecal coliforms or *E. coli,* for a period of time not longer than 120 minutes (after 2 hours of incubation, significant cell growth is observed). The detection step is performed by using a fluorimetric assay, known in literature, consisting in an extracellular enzymatic reaction of target enzymes β-galactosidase and β-glucuronidase with the relative fluorescent substrates methylumbelliferyl-β-D-galactoside (MUGal) and methylumbelliferyl-β-D-glucuronide (MUGlu). The hydrolysis reaction starts with the addition of the enzymatic substrate and organic solvent. The reaction is stopped by adding sodium or potassium hydroxide, which also amplify the fluorescence signal. There are no time limits for the hydrolysis, usually lasting from 5 to 120 minutes and preferably, but not necessarily, lasting 15 minutes. The lowest amount of cells detectable with the present method is about 10 cells/ sample. Hydrolysis is preferably shorter than 120 minutes, more preferably shorter than 60 minutes, still more preferably shorter than 30 minutes and most preferably shorter than 15 minutes. In any case the reaction time can be adjusted according to the number of cells. The method is so sensitive that it can detect down to 10 cells per sample and although exceeding, in such an eventuality, 20 minutes duration, will still be time-restrained (typically less than 3 hours) if compared to other known methods.

According to a preferred embodiment of the invention, the method for the detection of total coliforms is based on the use of the following reagents in agreement with the procedures hereby described; in a more preferred embodiment, the reagents can be assembled in kit form:
- **solution of enzymatic induction (Reagent A):** Reagent A comprises a water solution, pH between 2 and 10, including: a system buffer, like, for instance, sodium hydrogen phosphate (Na₂HPO₄) , potassium dihydrogen phosphate (KH₂PO₄), magnesium sulphate heptahydrate (MgSO₄·7H₂O), preferably but not necessarily at pH 7,2 (β-galactosidase) and pH 6,5 (β-glucuronidase); bivalent ions and, more specifically, the magnesium Mg⁺⁺, which are essential at a concentration, preferably but not necessarily, 0.5mM; at least an amino acid, preferably but not necessarily, tryptophan W, in a concentration up to 80mM; more preferably but not necessarily another amino acid, chosen between methionine M and threonine T, in a concentration up to 80mM and, still more preferably but not necessarily, also isoleucine I and leucine L, in a concentration up to 80mM; in general the use of the all natural 20 amino acids, promoters of the induction, is preferable but not necessary, at a concentration, preferably but not necessarily, 0.02mM each; an inducer of β-galactosidase or β-glucuronidase, like, for instance, isopropyl-β-D-thiogalactopyranoside or methyl-β-D-glucuronide and relative mixtures; a selective agent, acting as a membrane permeabilizer, like, for instance, sodium dodecyl sulphate; sodium chloride NaCl, preferably but not necessarily, at 0.01% (w/v); the solution may be sterilized, for instance, by filtering through a membrane filter with a pore size not superior to 0.45 µm, and can also eventually be in a lyophilized form;

- **solution of enzymatic substrate (Reagent B):** Reagent B comprises a solution of methylumbelliferyl-β-D-galactoside (MUGal) dissolved in dimethylsulfoxide and phosphate buffer, or a solution of methylumbelliferyl-β-D-glucuronide (MUGlu) dissolved in a solution of phosphate buffer/triton-x 99/1, or a mixture of both of them, preferably but not necessarily, characterized from the absence of 4-methylumbelliferone (MU), opportunely separated, for example, by passage on anionic column (i.e. resin Amberlite IRA-410 Sigma), to reduce the natural presence of 4-methylumbelliferone; the reagent can also eventually be in a lyophilized form;
- **solution of fluorescence amplification (Reagent C):** Reagent C comprises a sodium hydroxide (NaOH) solution in water, or any other base with the purpose of sensibly enhancing the natural fluorescence of 4-methylumbelliferone (up to 4 times), effect feasible at pH 11-12;
- **solvent:** the organic solvent (for example chloroform or other suitable solvent) causes the rupture of the bacterial membrane and, consequently, the cell lysis.

Moreover **Reagent A and Reagent B** can be in a lyophilized form and added to the original sample, without any sample pre-treatment, hence restoring an ideal environment of enzymatic induction and/or enzymatic hydrolysis, like hereby described.

The procedure according to the invention preferably comprises the following steps:
1. **sample preparation:**
   the sample can be analyzed directly, without any pre-treatment, by adding it directly to Reagent A; solid or semisolid samples can be extracted with a required volume of suitable buffer (preferably but not necessarily, a physiological solution or the formerly described phosphate buffer) and shortly processed like non pre-treated samples;
      alternatively, it is possible to separate the bacterial cells from the water sample by physical treatment, like filtering to concentrate bacteria on a membrane filter with a pore size not superior to 0.45 µm.
2. **enzyme induction:**
   as previously described, the sample can be:
      - directly added to Reagent A;
      - integrated with lyophilized Reagent A or lyophilized Reagent A + Reagent B, hence fitting the state of enzymatic induction;
      - filtered to concentrate bacteria on a membrane filter and the filter immersed in a minimum volume of Reagent A, enough to cover up the retained material; in all the embodiments the system "sample-Reagent A" is incubated between 30°C and 40°C for total coliforms, and between 30°C and 50°C for faecal coliforms or *E. coli,* for a period of time not longer than 120 minutes, for example at least 5 minutes, like aforesaid;
3. **expression of the activity of the induced enzyme through lysis of the cell membrane:**
   prior to, throughout or subsequent to the induction phase, Reagent B is added to the system "sample-Reagent A" (preferably but not necessarily 50µl/ml Reagent B and instantly following the induction period); Reagent B contains the fluorogenic substrate; after induction, the rupture of the bacterial membrane and, consequently, the cell lysis, is achieved by adding an organic solvent, for example chloroform or other suitable solvent; this allows the induced enzyme to exit the cell; the hydrolysis of the fluorogenic substrate takes place, with discharge of the fluorescent cleavage product; after vortexing, the sample is incubated at a temperature not higher than 50°C, for a time that can typically last between 5 and 15 minutes, for example 10 minutes; at the end of this period, the reaction is stopped by adding Reagent C up to pH 11-12.
4. **measurement:**
   the resulting solution is transferred to a quartz cuvette, and then placed in the fluorimeter provided with heating block; the excitation wavelength is set between 330 and 390 nm, the emission wavelength is set between 410 and 470 nm with slit widths between 2.5 and 20 nm; a blank reading needs to be performed by simulating the sample procedure, except for the inducing agents, in order to account for contaminating particles and background fluorescence; the control value must be subtracted from the sample result; in case of absence of interfering substances, it is also possible to achieve a control value just by simulating the sample analysis, except for the addition of the cells; operating on the field, having thus to deal with potential filter-retained interferents, the blank can be performed by omitting the step of cell lysis.
5. **evaluation of results:**
   a positive test result is defined as a statistically significant increase, when compared with control, in the fluorescence measurement, expressed in instrument-dependent arbitrary units; test positivity can be both qualitatively and quantitatively assessed; the use of a qualitative approach allows to differentiate total coliforms from faecal coliforms, according to the induction temperature, and faecal coliforms from *E*. *coli,* by means of their specific enzyme; a quantitative evaluation requires the elaboration of a fluorescence intensity calibration curve achieved by measuring samples containing known concentrations of target microbes (total coliforms, faecal coliforms or *E*. *coli*); a linear relation between fluorescence intensity and bacterial concentration can then be determined, allowing to calculate the number of cells contained in unknown samples; the calibration line is strictly dependent on the specific operating conditions; in order to provide reliable results, every little variation of parameters shall consequently be supported by the calculation of a fresh regression line.

Also contemplated as falling within the scope of the invention is an analysis kit comprising:
- Reagent A, Reagent B, Reagent C;
- instructions for use;
- suitable instrumentation and solvents.

The advantages of the new method include the following:
1. **the dramatic reduction of detection time:**
   the analysis is preferably shorter than 120 minutes, more preferably shorter than 60 minutes, still more preferably shorter than 30 minutes and most preferably shorter than 15 minutes; in any case, the reaction time can be adjusted according to the number of cells; the method is so sensitive that it can detect down to 10 cells per sample and, although exceeding, in such an eventuality, 20 minutes duration, it will still be time-restrained (typically less than 3 hours) if compared to other known methods, owing to the use of a new induction medium, able to stimulate, in absence of numerical cell growth, the production of detectable quantities of β-galactosidase and β-glucuronidase, even in samples containing a very small number of cells;
2. **the original use of a mixture of amino acids (" induction promoter "):** the mixture accounts for the aforementioned inductive properties, characteristic of the medium;
3. **the absence of nutritive substances in the induction medium preventing unwanted signal increase due to cell growth:**
   there is no need to wait for the exponential phase and, therefore, the measured enzymatic activity turns to be directly associated with the number of microorganisms originally contained in the sample; the variable connected to cell duplication shall not be considered, and a potential overestimation of the results during the fluorimetric measurement is thus circumvented;
4. **the presence of ion magnesium Mg₊₊:**
   by acting like cofactors for β-galactosidase and β-glucuronidase, ions magnesium are absolutely indispensable both in the enzymes induction and hydrolysis;
5. **the further increase of β-galactosidase activity as a consequence of the use of higher substrate concentration, achieved thanks to the solubilization in dimethylsulfoxide (Dmso) and potential purification on anionic column:**
   because of Dmso, the solubility of methylumbelliferyl-β-D-galactoside (MUGal) increases by 100 times; anionic resin helps remove free methylumbelliferone contaminating commercial methylumbelliferyl-β-D-galactoside (MUGal), resulting thus in a remarkable reduction of background fluorescence;
6. **improved detectability of the fluorophore due to extracellular enzyme hydrolysis:**
   the targeted enzymes reside inside the cell; chloroform leads to cell lysis and discharge of those intracellular induced enzymes; the enzymatic reaction with relative substrates, being everything in solution, is thus accelerated, and the free fluorophore can easily be determined spectrofluorimetrically.

The use of an "induction solution" for the detection of *E*. *coli* and total coliforms, object of the current invention, includes the following advantages:
1. the *reduction in detection time* (from 10 to 120 minutes), due to induction not being associated with cell growth, together with the *high sensitivity of the analysis,* due to induction being directly promoted from amino acids, which stimulate the production of a detectable amount of enzyme, even in a very small number of cells;
2. the *precision of the result* and the *linear correlation* between induced enzymatic activity and number of cells, due to *absence of cell proliferation;* the cell growth would actually introduce another variable in the correlation between the enzymatic activity and the original number of cells.

The induction solution, object of the present invention, was used to perform the analyses described in the following examples; a fluorimetric assay, based on the extracellular reaction of the enzymes with relative substrates methylumbelliferyl-β-D-galactoside (MUGal) and methylumbelliferyl-β-D-glucuronide (MUGlu), was selected to measure β-galactosidase and β-glucuronidase activity.

These and other objects of the invention will become more readily apparent from the following detailed description of several preferred embodiments of the invention. However, the examples set forth herein are in no way intended to limit the scope of the invention.

### Mode for the Invention

**Example 1- Induction of β-galactosidase activity on *E*. *coli* and *Enterococcus faecalis* and cell count when positive.**

### Sample preparation

Different samples in physiological solution (sodium chloride NaCl, 0.85% in water) containing *E. coli* cells (ATCC 25922) and *Enterococcus faecalis* cells (ATCC 29212) were variously diluted. For each sample, the cell number was verified by plate count method.

### Enzyme induction

33 µl of sample were added to 2315 µl of filter-sterilized (0.45 µm) induction solution, at pH 7.2, comprising sodium hydrogen phosphate (Na₂HPO₄) 47.7 mM, potassium dihydrogen phosphate (KH₂PO₄) 22 mM, magnesium sulphate heptahydrate (MgSO₄ ·7H₂O) 0.5 mM, 80µg of natural amino acids (alanine A, cysteine C, aspartic acid D, glutamic acid E, phenylalanine F, glycine G, histidine H, isoleucine I, lysine K, leucine L, methionine M, asparagine N, proline P, glutamine Q, arginine R, serine S, threonine T, valine V, tryptophan W, tyrosine Y, 4 µg each), 250 µg of sodium dodecyl sulphate and 125 µg of β-galactosidase inducer isopropyl-β-D-thiogalactopyranoside; the mixture was then incubated at 37°C for 75 minutes.

### Enzyme - substrate reaction

At the end of the induction time, 132 µl of methylumbelliferyl-β-D-galactoside (MUGal) in dimethylsulfoxide (1.5 mg/ml) and 20 µl of chloroform were added to the induction mixture; after vortexing, the hydrolysis mixture was incubated for 45 minutes at 37°C. The hydrolysis was then interrupted by adding 100 µl of sodium hydroxide (NaOH) 2 N in water.

### Measurement

The reading mixture was immediately transferred to a quartz cuvette and placed in the fluorimeter (Perkin-Elmer LS50B), with heating block set at 37°C; the excitation wavelength was set at 362 nm and the emission wavelength was set at 445 nm with slit widths at 2.5 nm. A blank reading needed to be performed by simulating the sample procedure, except for the addition of cells, being potential contaminating particles absent. *E. coli* count was deduced from a previously calculated regression line. **y=30.529x** *(y=cell number x=relative fluorescence units)*

**Table 1**

| Slit (2.5;2.5) | r.f.u. (tot) | r.f.u. (tot-B) | Cells (2ml) |
|---|---|---|---|
| Blank B | 47 | 0 | 0 |
| Sample 1 | 84 | 37 | 1,130 |
| Sample 2 | 116 | 69 | 2,107 |
| Sample 3 | 145 | 98 | 2,992 |
| Sample 4 | 179 | 132 | 4,030 |
| Sample 5 | 211 | 164 | 5,007 |
| Sample 6 | 246 | 199 | 6,075 |
| Sample 7 | 274 | 227 | 6,930 |
| Sample 8 | 308 | 261 | 7.968 |
| Sample 9 | 325 | 278 | 8,487 |
| Sample 10 | 357 | 310 | 9,464 |
| Enterococcus 1,2,3 | 47 | 0 | Not det. |

Samples 1-10 relative to *E*. *coli.*
r.f,u. = relative fluorescence units.
B = Blank (background fluorescence).

**Example 2- Induction of** β**-glucuronidase activity on *E. coli* and *Klebsiella pneumoniae* and cell count when positive.**

### Sample Preparation

Different samples in physiological solution (sodium chloride NaCl, 0.85% in water) containing *E*. *coli* cells (ATCC 25922) and *Klebsiella pneumoniae* cells (ATCC 13883) were variously diluted. For each sample, the cell number was verified by plate count method.

### Enzyme induction

33 µl of sample were added to 2315 µl of filter-sterilized (0.45 µm) induction solution, as described in Example 1, except for β-glucuronidase inducer, 1.25 mg of methyl-β-D-glucuronide; the mixture was then incubated at 44°C for 75 minutes.

### Enzyme - substrate reaction

At the end of the induction time, 132 µl of methylumbelliferyl-β-D-glucuronide (MUGlu) in phosphate buffer (sodium hydrogen phosphate (Na₂HPO₄) 47.7 mM, potassium dihydrogen phosphate (KH₂PO₄) 22 mM, magnesium sulphate heptahydrate (MgSO₄·7H₂O) 0.5 mM/Triton-X 99/1 (1.5 mg/ml) and 20 µl of chloroform were added to the induction mixture; after vortexing, the hydrolysis mixture was incubated for 45 minutes at 44°C. The hydrolysis was then interrupted by adding 100 µl of sodium hydroxide (NaOH) 2 N in water.

### Measurement

The reading mixture was immediately transferred to a quartz cuvette and placed in the fluorimeter (Perkin-Ehner LS50B), with heating block set at 44°C; the excitation wavelength was set at 362 nm and the emission wavelength was set at 445 nm with slit widths at 5.0 nm. A blank reading needed to be performed by simulating the sample procedure, except for the addition of cells, being potential contaminating particles absent. E. *coli* count was deduced from a previously calculated regression line. **y=30.993x** *(y=cell number x=relative fluorescence units)*

**Table 2**

| Slit (5;5) | r.f.u. (tot) | r.f.u. (tot-B) | Cells (2ml) |
|---|---|---|---|
| Blank B | 27 | 0 | 0 |
| Sample 1 | 84 | 14 | 434 |
| Sample 2 | 116 | 31 | 961 |
| Sample 3 | 145 | 48 | 1488 |
| Sample 4 | 179 | 61 | 1891 |
| Sample 5 | 211 | 77 | 2386 |
| Sample 6 | 246 | 89 | 2758 |
| Sample 7 | 274 | 115 | 3564 |
| Sample 8 | 308 | 127 | 3936 |
| Sample 9 | 325 | 149 | 4618 |
| Sample 10 | 357 | 147 | 4556 |
| Klebsiella 1,2,3 | 27 | 0 | Not det. |

Samples 1-10 relative to *E. coli.*
r.f.u. = relative fluorescence units.
B = Blank (background fluorescence).

**Example 3- Induction of β-galactosidase activity on *Klebsiella pneumoniae* and cell count when positive.**

### Sample preparation

Different samples in physiological solution (sodium chloride, 0.85% in water) containing *Klebsiella pneumoniae* cells (ATCC 13883) were variously diluted. Each cell number was verified by plate count method.

### Enzyme Induction and enzyme - substrate reaction

The enzyme induction and the expression of the enzymatic activity by cell lysis were performed according to example 1.

### Measurement

The measurement was performed according to Example 1 by using a previously calculated regression line. **y=60.063x** *(y=cell number x=relative fluorescence units).*

**Table 3**

| Slit (5;5) | r.f.u. (tot) | r.f.u. (tot-B) | Cells (2ml) |
|---|---|---|---|
| Blank B | 232 | 0 | 0 |
| Sample 1 | 257 | 25 | 1502 |
| Sample 2 | 305 | 73 | 4385 |
| Sample 3 | 323 | 91 | 5466 |
| Sample 4 | 329 | 97 | 5826 |
| Sample 5 | 401 | 169 | 10151 |
| Sample 6 | 427 | 195 | 11900 |
| Sample 7 | 448 | 216 | 12974 |
| Sample 8 | 523 | 291 | 17478 |
| Sample 9 | 526 | 294 | 17659 |

Samples 1-9 relative to *Klebsiella pneumoniae.*
r.f.u. = relative fluorescence units.
B = Blank (background fluorescence).

**Example 4 - Induction of β-galactosidase and β-glucuronidase activity on *E*. *coli* in absence of amino acids.**

Different samples in physiological solution (sodium chloride NaCl, 0.85% in water) containing *E. coli* cells (ATCC 25922) were variously diluted. For each sample, the cell number was verified by plate count method.

Enzyme induction, expression of the enzymatic activity by cell lysis and fluorimetric measurement were performed, except for the addition of the amino acids, according to Example 1 (β-galactosidase) and according to Example 2 (β-glucuronidase).

The results achieved from the fluorimetric assay show that, in absence of amino acids in the solution of induction, the cells do not produce the inducible enzymes in a detectable quantity.

## Claims

1. An induction solution which provides for rapid detection of coliforms, capable to induce, in absence of cell growth, the expression of inducible enzymes β-glucuronidase and β-galactosidase, comprising:
- one or more amino acids in such a quantity not to allow, between 0 and 120 minutes, a detectable cell growth of the coliforms in contact with; the amino acids concentration being up to 80 mM;
- a buffer system;
- a bivalent ion; and
- an enzyme inducer consisting of isopropyl-β-thiogalactopyranoside and/or methyl- β-D-glucuronide

2. A solution according to Claim 1, **characterized by** comprising all the twenty amino acids.

3. A solution according to Claim 1, **characterized by** comprising a combination of the following amino acids:
- tryptophane W;
- at least one between methionine M and threonine T;
- isoleucine I; and
- leucine L

4. A solution according to anyone of the previous Claims, **characterized in that** the bivalent ion is Mg⁺⁺ in a concentration up to 0.5 mM.

5. A solution according to anyone of the previous Claims, **characterized in that** the enzyme inducer isopropyl-β-thiogalactopyranoside is used in a concentration of about 0.2 mM and that the enzymatic inducer methyl- β-D-glucuronide is used in a concentration of about 2 mM.

6. A solution according to anyone of the previous Claims, **characterized by** comprising a selective agent acting as a membrane permeabilizer.

7. A solution according to Claim 6, **characterized in that** the selective agent acting is sodium dodecyl sulphate.

8. An analysis kit for the rapid detection of coliform cells comprising:
- an induction solution according to anyone of the previous Claims;
- a solution comprising a fluorescent substrate; and
- an organic solvent, capable to induce cell lysis.

9. A kit according to Claim 8, **characterized in that** said organic solvent is chloroform and/or triton-x.

10. A kit according to Claim 8 or 9, **characterized in that** the coliforms are selected among total coliforms, faecal coliforms, *E. coli.*

11. A kit according to anyone of the Claims 8 to 10, **characterized in that** the induction solution is filter-sterilized by passage through 0.45 µm or less pore size membrane filter and/or is in a lyophilized form.

12. A kit according to anyone of the Claims 8 to 11, **characterized in that** the fluorescent substrate is selected from the group consisting of methylumbelliferyl-β-D-galactoside, methylumbelliferyl-β-D-glucuronide and relative mixtures.

13. A kit according to anyone of the previous Claims 8 to 11, **characterized by** comprising a fluorescence amplifier constituted from a solution of a base capable to increase pH of the induction solution to about 11-12.

14. A process for the rapid detection of coliform bacteria, comprising the steps of:
- placing the sample to be analyzed in contact with an induction solution A, according to Claim 1 and incubating between 30 and 50°C;
- adding a fluorogenic substrate;
- rupturing the cells with any suitable lysis agent to release the induced enzyme;
- assisting hydrolysis of fluorogenic substrate with discharge of the corresponding fluorophore by incubating at less than 50°C, for a period of time depending on cell number;
- raising final mixture pH to about 11-12 with a basic solution, for example sodium hydroxide (NaOH) in water.

15. A process according to Claim 14, **characterized in that** the temperature is between 30 and 40°C for the detection of total coliforms and between 30 and 50°C for the detection of faecal coliforms or *E. coli.*

16. A process according to Claims 14 or 15 **characterized in that** the fluorophore concentration is determined by fluorimetric assay after extracellular hydrolysis reaction of target enzymes with relative substrates being, respectively, methylumbelliferyl-β-D-galactoside for β-galactosidase and methylumbelliferyl-β-D-glucuronide for β-glucuronidase.

17. A process according to anyone of the Claims 14 to 16, **characterized in that** the lysis agent is chloroform and/or triton-x.

18. A process according to anyone of the Claims 14 to 17, **characterized in that** the fluorescent substrate is methylumbelliferyl-β-D-galactoside in dimethylsulfoxide and/or methylumbelliferyl-β-D-glucuronide in water/triton-x.

19. A process according to anyone of the Claims 14 to 18, **characterized in that** the sample to be analyzed is pre-extracted with physiological saline or phosphate buffered saline solution.

20. A process according to Claim 19, **characterized in that** the sample is filtered on a 0.45 µm or less membrane filter and the filter is immersed in the induction solution.

21. A process according to anyone of the Claims 14 to 20, **characterized in that** the sample analysis is performed by fluorimetry, being the excitation wavelength set between 330 and 390 nm and the emission wavelength set between 410 and 470 nm with slit widths between 2.5 and 20 nm.

## Patentansprüche

1. Induktionslösung, die einen raschen Nachweis von Coliformen ermöglicht, die fähig sind, in Abwesenheit von Zellwachstum die Expression der induzierbaren Enzyme β-Glucuronidase and β-Galactosidase zu induzieren, umfassend:
eine oder mehrere Aminosäure in einer solchen Menge, die in einem Zeitraum von 0 bis 120 Minuten ein nachweisbares Zellwachstum der Coliformen im Kontakt dazu nicht erlaubt; wobei die Aminosäurekonzentration bis zu 80 mM ist;
- ein Puffersystem;
- ein bivalentes Ion; und
- einen Enzyminduktor bestehend aus Isopropyl-β-thiogalactopyranosid und/oder Methyl-β-D-glucuronid.

2. Lösung nach Anspruch 1, **dadurch gekennzeichnet, dass** sie alle zwanzig Aminosäuren umfasst.

3. Lösung nach Anspruch 1, **dadurch gekennzeichnet, dass** sie eine Kombination der folgenden Aminosäuren umfasst:
- Tryptophan W;
- mindestens eines von Methionin M und Threonin T;
- Isoleucin I; und
- Leucin L.

4. Lösung nach einem der vorangehenden Ansprüche, **dadurch gekennzeichnet, dass** das bivalente Ion Mg⁺⁺ in einer Konzentration bis zu 0,5 mM ist.

5. Lösung nach einem der vorangehenden Ansprüche, **dadurch gekennzeichnet, dass** der Enzyminduktor Isopropyl-β-thiogalactopyranosid in einer Konzentration von etwa 0,2 mM verwendet wird und dass der enzymatische Induktor Methyl-β-D-glucuronid in einer Konzentration von etwa 2 mM verwendet wird.

6. Lösung nach einem der vorangehenden Ansprüche, **dadurch gekennzeichnet, dass** sie ein als Membranpermeabilisator wirkendes Selektionsagens umfasst.

7. Lösung nach Anspruch 6, **dadurch gekennzeichnet, dass** das wirksame Selektionsagens Natriumdodecylsulfat ist.

8. Analysekit zum raschen Nachweis von coliformen Zellen umfassend:
- eine Induktionslösung nach einem der vorangehenden Ansprüche;
- eine ein Fluoreszenzsubstrat umfassende Lösung; und
- ein zur Induzierung von Zelllyse fähiges organisches Lösungsmittel.

9. Kit nach Anspruch 8, **dadurch gekennzeichnet, dass** das organische Lösungsmittel Chloroform und/oder Triton-X ist.

10. Kit nach Anspruch 8 oder 9, **dadurch gekennzeichnet, dass** die Coliformen aus allen Coliformen, faekalen Coliformen, *E. coli* ausgewählt sind.

11. Kit nach einem der Ansprüche 8 bis 10, **dadurch gekennzeichnet, dass** die Induktionslösung mittels Passage durch einen Membranfilter mit einer Porengröße von 0,45 µm oder kleiner filtersterilisiert und/oder in lyophilisierter Form ist.

12. Kit nach einem der Ansprüche 8 bis 11, **dadurch gekennzeichnet, dass** das Fluoreszenzsubstrat ausgewählt ist aus der Gruppe bestehend aus Methylumbelliferyl-β-D-galactosid, Methylumbelliferyl-β-D-glucuronid und Gemischen daraus.

13. Kit nach einem der vorangehenden Ansprüche 8 bis 11, **dadurch gekennzeichnet, dass** er einen Fluoreszenzverstärker umfasst, der aus einer Lösung einer Base besteht, die den pH-Wert der Induktionslösung auf etwa 11-12 erhöhen kann.

14. Verfahren zum raschen Nachweis von coliformen Bakterien, umfassend die Schritte:
- Inkontaktbringen der zu analysierenden Probe mit einer Induktionslösung A nach Anspruch 1 und Inkubieren zwischen 30 und 50°C;
- Hinzufügen eines fluorogenen Substrats;
- Aufbrechen der Zellen mit einem beliebigen Lysemittel, um das induzierte Enzym freizusetzen;
- Unterstützen der Hydrolyse des fluorogenen Substrats mit Freisetzung des ensprechenden Fluorophors durch Inkubation bei unter 50°C über einen von der Zellanzahl abhängigen Zeitraum;
- Erhöhen des endgültigen pH-Wertes des Gemischs auf etwa 11-12 mit einer alkalischen Lösung, zum Beispiel Natriumhydroxid (NaOH) in Wasser.

15. Verfahren nach Anspruch 14, **dadurch gekennzeichnet, dass** die Temperatur zum Nachweis aller Coliformen zwischen 30 und 40°C und zum Nachweis der faekalen Coliformen oder von *E. coli* zwischen 30 und 50°C ist.

16. Verfahren nach Anspruch 14 oder 15, **dadurch gekennzeichnet, dass** die Fluorophorkonzentration nach der extrazellulären Hydrolysereaktion von Zielenzymen mit den entsprechenden Substraten mittels fluorometrischen Assays bestimmt wird, wobei die Substrate für β-Galactosidase Methylumbelliferyl-β-D-galactosid und für β-Glucuronidase Methylumbelliferyl-β-D-glucuronid sind.

17. Verfahren nach einem der Ansprüche 14 bis 16, **dadurch gekennzeichnet, dass** das Lysemittel Chloroform und/oder Triton-X ist.

18. Verfahren nach einem der Ansprüche 14 bis 17, **dadurch gekennzeichnet, dass** das Fluoroszenzsubstrat Methylumbelliferyl-β-D-galactosid in Dimethylsulfoxid und/oder Methylumbelliferyl-β-D-glucuronid in Wasser/Triton-X ist.

19. Verfahren nach einem der Ansprüche 14 bis 18, **dadurch gekennzeichnet, dass** die zu analysierende Probe mit physiologischer Kochsalzlösung oder Phosphat-gepufferter Kochsalzlösung vorextrahiert wird.

20. Verfahren nach Anspruch 19, **dadurch gekennzeichnet** das die Probe durch einen Membranfilter mit einer Porengröße von 0,45 µm oder kleiner gefiltert wird und der Filter in die Induktionslösung eingetaucht wird.

21. Verfahren nach einem der Ansprüche 14 bis 20, **dadurch gekennzeichnet, dass** die Probenanalyse mittels Fluorometrie durchgeführt wird, wobei die Anregungswellenlänge zwischen 330 und 390 nm eingestellt ist und die Emissionswellenlänge zwischen 410 und 470 nm mit Schlitzweiten zwischen 2,5 und 20 nm eingestellt ist.

## Revendications

1. Solution d'induction qui permet de détecter rapidement des coliformes, capable d'induire, en l'absence de croissance cellulaire, l'expression d'enzymes inductives β-glucuronidase et β-galactosidase, comprenant :
- un ou plusieurs acides aminés en une proportion telle qu'il ne peut pas y avoir, entre 0 et 120 minutes, de croissance cellulaire détectable des coliformes en contact avec ces derniers ; la concentration des acides aminés allant jusqu'à 80 mM ;
- un système tampon ;
- un ion bivalent ; et
- un inducteur enzymatique consistant en β-thiogalactopyranoside d'isopropyle et/ou méthyl-β-D-glucuronide.

2. Solution selon la revendication 1, **caractérisée par** le fait de comprendre l'ensemble des vingt acides aminés.

3. Solution selon la revendication 1, **caractérisée par** le fait de comprendre une combinaison des acides aminés suivants :
- tryptophane W ;
- au moins l'une parmi la méthionine M et la thréonine T ;
- l'isoleucine I ; et
- la leucine L.

4. Solution selon l'une quelconque des revendications précédentes, **caractérisée en ce que** l'ion bivalent est du Mg⁺⁺ en une concentration allant jusqu'à 0,5 mM.

5. Solution selon l'une quelconque des revendications précédentes, **caractérisée en ce que** l'inducteur enzymatique β-thiogalactopyranoside d'isopropyle est utilisé en une concentration allant jusqu'à environ 0,2 mM et **en ce que** l'inducteur enzymatique méthyl-β-D-glucuronide est utilisé en une concentration d'environ 2 mM.

6. Solution selon l'une quelconque des revendications précédentes, **caractérisée par** le fait de comprendre un agent sélectif faisant office de perméabilisant membranaire.

7. Solution selon la revendication 6, **caractérisée en ce que** l'agent sélectif agissant est du dodécylsulfate de sodium.

8. Kit d'analyse pour détecter rapidement des cellules coliformes comprenant :
- une solution d'induction selon l'une quelconque des revendications précédentes ;
- une solution comprenant un substrat fluorescent ; et
- un solvant organique capable d'induire une lyse cellulaire.

9. Kit selon la revendication 8, **caractérisé en ce que** ledit solvant organique est du chloroforme et/ou le triton-x.

10. Kit selon la revendication 8 ou 9, **caractérisé en ce que** les coliformes sont sélectionnés parmi des coliformes totaux, des coliformes d'origine fécale, E. Coli.

11. Kit selon l'une quelconque des revendications 8 à 10, **caractérisé en ce que** la solution d'induction est stérilisée par filtration en la faisant passer à travers un filtre membranaire dont la taille des pores est de 0,45 µm ou moins et/ou elle se trouve sous forme lyophilisée.

12. Kit selon l'une quelconque des revendications 8 à 11, **caractérisé en ce que** le substrat fluorescent est sélectionné du groupe constitué par du méthylumbelliféryl-β-D-galactoside, méthylumbelliféryl-β-D-glucuronide et de leurs mélanges.

13. Kit selon l'une quelconque des revendications 8 à 11 précédentes, **caractérisé par** le fait de comprendre un amplificateur de fluorescence constitué à partir d'une solution d'une base capable d'accroître le pH de la solution d'induction jusqu'à environ 11-12.

14. Procédé destiné à détecter rapidement des bactéries coliformes, comprenant les étapes qui consistent à :
- placer l'échantillon à analyser en contact avec une solution d'induction A, selon la revendication 1 et incuber entre 30 à 50°C ;
- ajouter un substrat fluorogénique ;
- casser les cellules avec tout agent de lyse approprié afin de libérer l'enzyme induite ;
- assister l'hydrolyse du substrat fluorogénique par une décharge du fluorophore correspondant en faisant une incubation à une température inférieure à 50°C pendant une durée qui dépend du nombre de cellules ;
- relever le pH du mélange final jusqu'à environ 11-12 à l'aide d'une solution basique, par exemple, de l'hydroxyde de sodium (NaOH) dans l'eau.

15. Procédé selon la revendication 14, **caractérisé en ce que** la température se trouve entre environ 30 et 40°C pour la détection de coliformes totaux et entre 30 et 50°C pour la détection de coliformes d'origine fécale ou d'*E. Coli.*

16. Procédé selon les revendications 14 ou 15 **caractérisé en ce que** la concentration en fluorophore est déterminée par dosage fluorimétrique après réaction d'hydrolyse extracellulaire d'enzymes cibles avec des substrats associés qui sont, respectivement, du méthylumbelliféryl-β-D-galactoside pour β-galactosidase et du méthylumbelliféryl-β-D-glucuronide pour β-glucuronidase.

17. Procédé selon l'une quelconque des revendications 14 à 16, **caractérisé en ce que** l'agent de lyse est du chloroforme et/ou du triton-x.

18. Procédé selon l'une quelconque des revendications 14 à 17, **caractérisé en ce que** le substrat fluorescent est du méthylumbelliféryl-β-D-galactoside dans du sulfoxyde de diméthyle et/ou méthylumbelliféryl-β-D-glucuronide dans de l'eau/triton-x.

19. Procédé selon l'une quelconque des revendications 14 à 18, **caractérisé en ce que** l'échantillon à analyser est pré-extrait à l'aide d'une solution saline tamponnée par les phosphates ou physiologique.

20. Procédé selon la revendication 19, **caractérisé en ce que** l'échantillon est filtré sur un filtre membranaire de 0,45 µm ou moins et le filtre est immergé dans la solution d'induction.

21. Procédé selon l'une quelconque des revendications 14 à 20, **caractérisé en ce que** l'analyse de l'échantillon est réalisée par fluorimétrie, la longueur d'onde d'excitation étant réglée entre 330 et 390 nm et la longueur d'onde d'émission étant réglée entre 410 et 470 nm et les largeurs de fentes se trouvant entre 2,5 et 20 nm.
